# EUROPÄISCHE PATENTSCHRIFT

(11) **EP 0 193 957 B1**
(45) Veröffentlichungstag und Bekanntmachung des Hinweises auf die Patenterteilung: **06.11.1996**
(21) Anmeldenummer: 86102979.1
(22) Anmeldetag: 06.03.1986
(51) Int. Cl.: F22B 1/00, A61L 2/06

(54) **Verfahren zur Dampferzeugung für die Dampfsterilisation**
Steam generation method for steam sterilization
Méthode de production de vapeur pour la stérilisation à vapeur

(30) Priorität: 07.03.1985 DE 3508043
(43) Veröffentlichungstag der Anmeldung: 10.09.1986
(73) Patentinhaber: Fr. Kammerer GmbH, D-75181 Pforzheim (DE); DEUTSCHE FORSCHUNGSANSTALT FÜR LUFT- UND RAUMFAHRT e.V., 53111 Bonn (DE)
(72) Erfinder: Sternfeld, Hans Joachim, Prof. Dr.-Ing. Dipl.-Ing., D-7109 Jagsthausen (DE); Wolfmüller, Karlheinz, Dr. Dipl.-Ing., D-7519 Eppingen-Adelshofen (DE); Froese, Rudhardt, D-7542 Schömberg (DE); Paulus, Manfred, Dr. Ing. Dipl.-Phys., D-7530 Pforzheim 14 (DE)
(74) Vertreter: Twelmeier, Ulrich, Dipl.Phys.

(56) Entgegenhaltungen:
- DE-B- 1 301 821
- DE-C- 319 821
- DE-C- 815 534
- DE-C- 819 129
- GB-A- 1 048 276
- GB-A- 1 449 483

## Beschreibung

Die wesentlichsten Anwendungsgebiete für die Sterilisation mit Dampf sind die Medizintechnik, die Pharmazie, die Lebensmitteltechnik und die Biotechnik.

In der Medizintechnik z.B. werden Dampfsterilisatoren in vielfältiger Weise eingesetzt. Dementsprechend unterschiedlich sind auch ihre Baugrößen. Die kleinsten Geräte dienen der Sterilisation von Instrumentarien in Arzt- und Zahnarztpraxen, es handelt sich dabei in der Regel um kleine elektrisch beheizte Dampferzeuger (Autoklaven). In Kliniken sind größere Anlagen für die Sterilisation von medizinischen Gerätschaften aus unterschiedlichen Materialien wie Glas, Metall, Gummi, Keramik, Kunststoff oder Zellstoff und von Textilien wie Verbrauchsmaterialien oder Wäsche installiert. Es handelt sich dabei um Dampfsterilisatoren, welche durch fest installierte Dampfringleitungen mit einem zentralen Dampferzeuger verbunden sind oder durch separate elektrisch beheizte Dampferzeuger gespeist werden.

Da die zu sterilisierenden Gegenstände aus sehr unterschiedlichen Werkstoffen bestehen, die sehr unterschiedliche Temperaturbelastbarkeit aufweisen, muss diesen Gegebenheiten bei der Festlegung der Sterilisationsbedingungen Rechnung getragen werden. Bei herkömmlichen Autoklaven für Sterilisationszwecke können nur Dampfzustände verwirklicht werden, die an die Siedelinie im Zustandsdiagramm des Wassers gebunden sind. Weil bei der Festlegung der Sterilisationsbedingungen von den zur Verfügung stehenden Dampfzuständen ausgegangen werden muss und diese im Stand der Technik nur in begrenztem Umfang veränderbar sind, sind der Wahl der Sterilisationsbedingungen dort enge Grenzen gesetzt.

Die Sterilisationsbedingungen können nur über die Wahl der Behandlungsdauer und die Änderung der Heizleistung beeinflußt werden, wobei es ein Nachteil ist, dass eine Temperaturerhöhung stets mit einer Drucksteigerung im Autoklaven erkauft wird, welche die Temperaturerhöhung begrenzt. Darüberhinaus braucht die Dampferzeugung geraume Zeit.

Aus der DE-C-815 534 ist eine Vorrichtung zum Sterilisieren bekannt, welche eine abgeschlossene Sterilisierkammer hat, welche von einem angeschlossenen Dampferzeuger mit Dampf versorgt wird, in welchem der Dampf nicht wie bei den allgemein üblichen Autoklaven durch direktes elektrisches Beheizen von Wasser erzeugt wird, sondern dadurch, dass ein metallischer Wärmespeicher über längere Zeit vorgeheizt und dann nach Bedarf mit Wasser in Berührung gebracht wird, welches dann rasch verdampft. Die Bereitstellung von Dampf ist dadurch zwar kurzfristig möglich, aber erst, nachdem das Gerät geraume Zeit vorgeheizt wurde, und nur solange der Wärmevorrat nicht verbraucht ist. Die Sterilisiervorrichtung ist deshalb nicht jederzeit verfügbar, und der Dampfzustand hängt von der Temperatur und Wärmekapazität des Speichers und vom Wärmeverbrauch ab, kann also nicht willkürlich gewählt werden.

Hinzu kommt, dass ebenso wie bei herkömmlichen Autoklaven nur chargenweise sterilisiert werden kann und die Sterilisierkammer erst geöffnet werden kann, nachdem durch Abkühlen oder Dampfablassen der Überdruck abgebaut wurde.

Die am Beispiel der Sterilisation in der Medizintechnik erwähnten Nachteile gelten sinngemäss auch für die Sterilisation im Bereich der Pharmazie, im Bereich der Lebensmitteltechnik und im Bereich der Biotechnik, wo es z.B. um die Sterilisation von Bioreaktoren, Fermentern und Peripheriegeräten geht.

Der Erfindung liegt die Aufgabe zugrunde, ein Verfahren für die Dampfsterilisation anzugeben, mit welchem ohne die genannten, sich aus der Autoklaviertechnik ergebenden Beschränkungen mit weniger Zeitaufwand sterilisiert werden kann.

Diese Aufgabe wird gelöst durch ein Verfahren mit den im Anspruch 1 angegebenen Merkmalen. Vorteilhafte Weiterbildungen der Erfindung sind Gegenstand der Unteransprüche.

Die Erfindung wendet ein Verfahren zur Dampferzeugung an, welches ursprünglich für die Anwendung im Bereich der Antriebstechnik und der Kraftwerkstechnik, z.B. als Energieerzeugungsaggregat für die Deckung des Spitzenbedarfs in Kraftwerken, konzipiert wurde (DE-PS 1 301 821 und DE-PS 2 933 932). Bei den daraus bekannten Dampferzeugern werden zur Erzeugung von Wasserdampf Wasserstoffgas und Sauerstoffgas durch einen Einspritzkopf in eine Brennkammer eingeleitet und darin verbrannt, und in die heißen Verbrennungsgase wird zusätzlich Wasser aus Rohrleitungen, welche in die Brennkammer einmünden, eingespritzt. Im unmittelbaren Kontakt mit den heißen Verbrennungsgasen verdampft das eingespritzte Wasser und der entstehende Wasserdampf wird durch den in der Brennkammer herrschenden hohen Druck aus der Brennkammer ausgetrieben.

Nach diesem Verfahren kann Wasserdampf erzeugt werden, dessen Zustand auf oder oberhalb der Siedelinie im Zustandsdiagramm des Wassers liegt. Der Dampfzustand hängt vom Mengenverhältnis des in die Brennkammer eingeführten Wasserstoff/Sauerstoffgemisches einerseits zur eingeführten Wassermenge andererseits ab; dieses kann über regelbare Ventile in den jeweiligen Zuleitungen schnell und ohne Schwierigkeiten verändert werden, sodass auch der Dampfzustand des auf diese Weise erzeugten Dampfes in weiten Grenzen nahezu verzögerungsfrei verändert werden kann.

Ein weiterer gewichtiger Vorteil der Erfindung liegt darin, dass man nach dem erfindungsgemäßen Verfahren praktisch ohne Anlaufzeit, nämlich innerhalb etwa einer Sekunde, Dampf in dem jeweils geforderten Zustand bereitstellen kann. Ein längeres Aufheizen des Dampferzeugers, welches man von Dampferzeugern für Sterilisatoren nach dem Stand der Technik gewohnt ist, entfällt vollständig.

Für einen erfindungsgemäß arbeitenden Dampferzeuger benötigt man allenfalls in sehr geringem Umfang elektrische Energie, nämlich für seine Steuerung, aber nicht für die eigentliche Dampferzeugung selbst. Anders als herkömmliche Dampfsterilisatoren können Sterilisatoren mit erfindungsgemäß arbeitenden Dampferzeugern auch in solchen Regionen betrieben werden, wo die Bereitstellung elektrischer Energie für die Dampferzeugung nicht gewährleistet ist.

Erfindungsgemäß arbeitende Dampferzeuger sind darüberhinaus ausserordentlich leistungsfähig. Schon bei kleinsten Abmessungen können große Mengen Dampf erzeugt werden. Dieses und die rasche Verfügbarkeit von Dampf in vorwählbarem Zustand machen das erfindungsgemäße Verfahren besonders geeignet für einen dezentralen Einsatz sowie insbesondere auch für eine nicht an feste Orte gebundene, sondern mobile Verwendung an unterschiedlichen Einsatzorten.

Als Dampf für die Sterilisation verwendet man in der Regel reinen Wasserdampf, der aus destilliertem oder deionisiertem Wasser entsteht. Für besondere Anwendungen können aber dem Wasserdampf auch Dämpfe sterilisierend wirkender Flüssigkeiten zugegeben werden. Ausserdem ist es möglich, bereits dem Wasser, welches verdampft werden soll, eine andere sterilisierend wirkende Substanz, z.B. Formaldehyd, zuzusetzen und die zusammengesetzte Flüssigkeit zur Dampferzeugung zu verwenden.

Als Brennstoff verwendet man Wasserstoff, den man mit Sauerstoff oder Luft zu Wasserdampf verbrennt. Verwendet man als Brenngase Wasserstoff und Sauerstoff in stöchiometrischem Verhältnis, dann entsteht voll kondensierbarer Wasserdampf.

Man kann den Wasserstoff und den Sauerstoff auch in nicht-stöchiometrischem Verhältnis einsetzen, insbesondere den Wasserstoff unter Sauerstoffüberschuss oder in Luft verbrennen. In einem solchen Fall enthält der Wasserdampf nicht-kondensierbare Anteile.

Mit einem erfindungsgemäß arbeitenden Dampferzeuger kann man in einem Sterilisator die Sterilisationsbedingungen, die durch den Dampfzustand, gekennzeichnet durch den Druck, die Temperatur und die Dampffeuchte, sowie durch die Dampferzeugungsrate (in der Zeiteinheit erzeugte Dampfmenge ) gegeben sind, weitgehend beliebig einstellen. Zu diesem Zweck ist es lediglich erforderlich, die Zufuhr des Wasserstoffes und des Sauerstoffes und/oder die Zufuhr des zu verdampfenden Wassers zur Erzielung eines vorgewählten Dampfzustandes zu steuern oder zu regeln. Eine selbsttätige Regelung ist möglich, wenn man im Sterilisator Meßfühler vorsieht, die den Dampfzustand erfassen und an ein Steuergerät melden, welches die Zufuhr des Wasserstoffs und des Sauerstoffs und/oder des zu verdampfenden Wassers über regelbare Ventile so steuert, dass sich ein vorgewählter Dampfzustand einstellt. In Weiterbildung des Verfahrens ist es darüberhinaus möglich, während des Sterilisationsvorganges nicht nur einen gleich bleibenden Dampfzustand einzustellen, sondern in optimaler Anpassung an das zu sterilisierende Gut den Dampfzustand gemäß einem vorgegebenen Programm zu ändern, welches dem im Regelkreis vorgesehenen Steuergerät die aktuellen, sich zeitlich ändernden Sollwerte des Dampfzustandes vorgibt.

Eine derartige Steuerung des Dampfzustandes wird in Anwendung der Erfindung besonders leicht gemacht, weil ein erfindungsgemäß arbeitender Dampferzeuger sich praktisch verzögerungsfrei steuern läßt.

Ausführungsbeispiele der Erfindung sind schematisch in den beigefügten Zeichnungen dargestellt.
- Figur 1: zeigt in Blockdarstellung die Verwendung eines Dampferzeugers für die Sterilisation eines Behälters, und die
- Figur 2: zeigt in Blockdarstellung die Verwendung mehrerer Dampferzeuger für die Dampfsterilisation mehrerer Kammern einer Anlage.

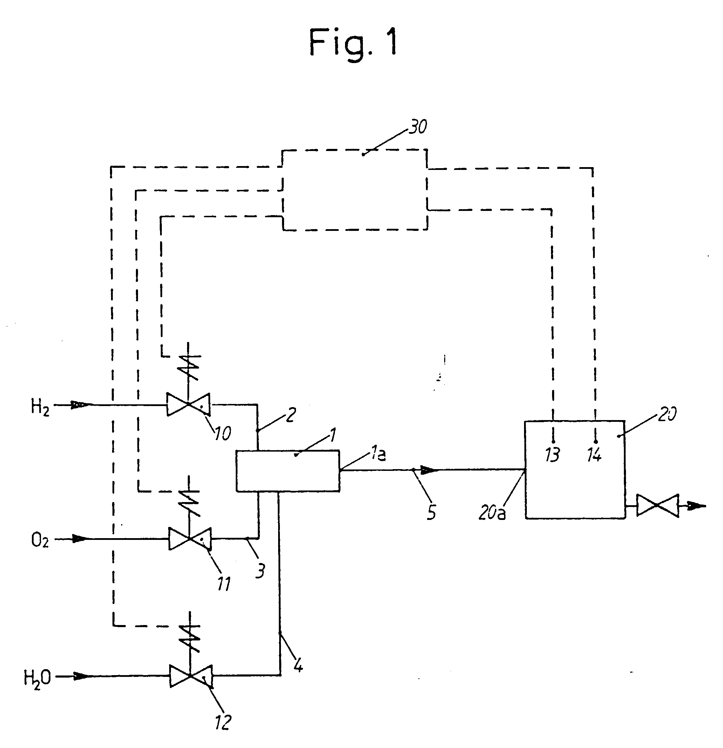

Figur 1 zeigt einen Dampferzeuger 1, in welchen durch eine erste Zuleitung 2 Wasserstoffgas, durch eine zweite Zuleitung 3 Sauerstoffgas und durch eine dritte Zuleitung 4 Wasser eingeleitet wird. Der Wasserstoff und der Sauerstoff werden im Dampferzeuger 1 zu Wasserdampf verbrannt und in diesen sehr heißen Wasserdampf wird das durch die Zuleitung 4 herangeführte Wasser eingebracht; vorzugsweise wird das Wasser möglichst fein und gleichmäßig versprüht, sodass es im unmittelbaren Kontakt mit dem heißen, aus der Verbrennung herrührenden Wasserdampf praktisch sofort verdampft.

Der auf diese Weise erzeugte Wasserdampf bewirkt im Dampferzeuger 1 einen gegenüber dem Fall ohne Reaktion erhöhten Druck und strömt durch eine Auslaßöffnung 1a aus dem Dampferzeuger heraus in einen Behälter 20, in welchem sich das zu sterilisierende Gut befindet.

Die Verbindung zwischen dem Dampferzeuger 1 und dem Behälter 20 kann durch eine Verbindungsleitung 5 gebildet sein, es ist aber auch durchaus möglich, den Auslaß la des Dampferzeugers unmittelbar am Einlaß 20a des Behälters 20 anzubringen.

Der Dampferzeuger 1 wird vorzugsweise aus Druckbehältern, die die Gase Wasserstoff und Sauerstoff getrennt enthalten, gespeist. Der Druck beider Gase wird über nicht dargestellte Reduzierventile auf den jeweils benötigten Vordruck eingestellt. In den Zuleitungen 2 und 3 liegen regelbare Ventile 10 und 11, durch welche sich der Zustrom von Wasserstoff und Sauerstoff in den Dampferzeuger 1 willkürlich dosieren läßt. Zweckmäßigerweise führt man den Wasserstoff und den Sauerstoff stets in stöchiometrischem Verhältnis zu. Im Dampferzeuger 1 werden der Wasserstoff und der Sauerstoff kontrolliert verbrannt. Die Verbrennung kann durch eine elektrische Zündung oder katalytisch eingeleitet werden.

Das Einbringen des durch die Leitung 4 zugeführten Wassers kann grundsätzlich auf beliebige Weise erfolgen, insbesondere durch Versprühen. Die Wassermenge, welche in der Zeiteinheit eingebracht wird, richtet sich nach dem geforderten Dampfzustand und wird mittels eines Regelorgans 12, z.B. eines Ventils, eingestellt, welches in der Zuleitung 4 liegt.

Die Steuerung des Dampferzeugers zur Abgabe einer bestimmten Dampfmenge sowie zur Einstellung eines vorgegebenen Dampfzustandes im Behälter 20 kann durch eine selbsttätige Regelung erfolgen. Zu diesem Zweck können im Behälter 20 Meßfühler 13 und 14 vorgesehen sein, die bestimmte physikalische Meßgrößen des Dampfes, insbesondere die Temperatur und den Druck erfassen. Die Meßfühler 13 und 14 übermitteln ihre Meßsignale an ein zentrales Regelgerät 30, in welchem die Sollwerte der von Meßfühlern 13 und 14 zu bestimmenden Meßgrößen vorgegeben sind und mit den Istwerten verglichen werden. Entsprechend den festgestellten Abweichungen der Istwerte von den Sollwerten steuert das Regelgerät 30 die Massenströme über die bereits genannten Regelorgane 10, 11 und 12. Veränderungen des Betriebszustandes des Dampferzeugers können in sehr kurzen Zeiten von weniger als einer Sekunde eingestellt werden. Dadurch ist es möglich, den Dampfzustand ausserordentlich rasch zu verändern und der Sterilisationsaufgabe optimal-anzupassen. Die für einen optimalen Sterilisationsablauf vorgegebenen zeitlichen Zustandsänderungen des Dampfes können dem Regelgerät 30 über programmierbare Steuerungen (z.B. über EPROMs) vorgegeben werden.

Der erfindungsgemäß arbeitende Dampferzeuger zeichnet sich gegenüber den herkömmlichen, elektrisch beheizten Dampferzeugern für Sterilisationszwecke dadurch aus, dass nach getrennter und unabhängiger Vorgabe von Druck und Temperatur Dampfzustände in weiten Grenzen des gesättigten und überhitzten Dampfzustandsbereiches realisiert werden können. Die Parameter des Dampfes aus herkömmlichen Dampferzeugern sind dagegen an die Siedelinie im Zustandsdiagramm des Wassers gebunden.

Damit ist erstmals die Möglichkeit gegeben, Sterilisationsprozesse im Hinblick auf die beste Sterilisationswirkung mit minimalem Zeitaufwand zu optimieren, ohne durch die Bauart des Dampferzeugers bereits im Voraus begrenzt zu sein.

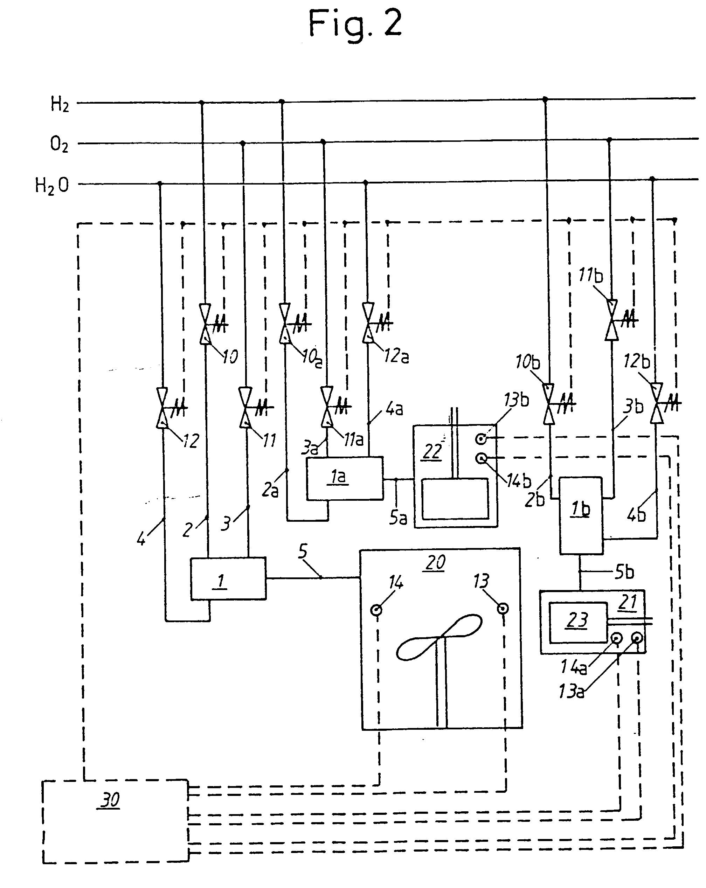

Im zweiten Ausführungsbeispiel (Fig. 2) sind Teile, die Teilen im ersten Ausführunsbeispiel entsprechen, mit denselben Bezugszahlen wie in Fig. 1 bezeichnet. Figur 2 zeigt eine Anlage mit mehreren Kammern 20, 21 und 22, in welche zu Sterilisationszwecken Dampf eingeleitet werden soll. Bei der Anlage kann es sich z.B. um eine Fermenteranlage in der Biotechnik handeln. In diesem Fall wäre der Behälter 20 der eigentliche Fermentationsbehälter, die Kammer 21 z.B. eine Schleuse für eine auswechselbare Sonde 23 zum Einführen in die Kammer 20, und die Kammer 22 wäre beispielsweise Teil einer Chargiereinrichtung, welche für das Beladen des Fermentationsbehälters 20 benötigt wird. Entsprechend dem Ablauf des Fermentationsprozesses müssen die Kammern 20, 21 und 22 zu unterschiedlichen Zeitpunkten sterilisiert werden. Jede dieser Kammern ist deshalb über eine Zuleitung 5 bzw. 5a bzw. 5b mit einem eigenen Dampferzeuger 1 bzw. la bzw. 1b verbunden. Jeder dieser Dampferzeuger ist für sich ebenso wie der Dampferzeuger im ersten Ausführungsbeispiel über Zuleitungen 2, 3, 4 bzw. 2a, 3a, 4a bzw. 2b, 3b, 4b, in welchen regelbare Ventile 10,11,12 bzw. 10a,11a,12a bzw. 10b,11b,12b liegen, mit einer Quelle für Wasserstoffgas, für Sauerstoffgas sowie für Wasser verbunden.

Da erfindungsgemäß arbeitende Dampferzeuger über einen extrem breiten Regelbereich der Dampfzustandsbedingungen verfügen, können alle drei Dampferzeuger dieselbe Baugröße haben. Die Steuerung der Dampfzustandsbedingungen kann wie im ersten Ausführungsbeispiel durch ein gemeinsames Regelgerät 30 erfolgen, welches über Meßfühler 13 und 14 in der Kammer 20, durch Meßfühler 13a und 14a in der Kammer 21 sowie durch Meßfühler 13b und 14b in der Kammer 22 die Dampfzustandsbedingungen überwacht. Die Steuerung der Ventile 10, 11, 12 bzw. 10a, 11a, 12a bzw. 10b, 11b, 12b erfolgt zweckmäßigerweise getrennt, sodass in jeder Kammer 20, 21 und 22 zum jeweils richtigen Zeitpunkt der geeignete Dampfzustand eingestellt wird. In einfachen Anwendungen könnten die verschiedenen Ventile aber auch gemeinsam gesteuert werden.

Durch die gezielte Anpassung und Veränderung der Dampfparameter wie Druck, Temperatur, Dampffeuchte und Dampfmenge an die momentanen Bedingungen im Fermenter können die Gesamtsterilisationszeiten im Vergleich zum Stand der Technik verkürzt und die Sterilisationswirkung verbessert werden.

Weil die Anwendung des erfindungsgemäßen Verfahrens mit kleinen, preiswerten Dampferzeugern möglich ist, können ohne weiteres für eine Fermenteranlage mehrere Dampferzeuger verwendet und getrennt gesteuert werden. Neben dem eigentlichen Fermentationsraum können andere Kammern der Anlage, z.B. Schleusenkammern, Einführungskammern von Elektroden oder Meßfühlern oder Kammern für die Probenentnahme, mit gesonderten, fest installierten Dampferzeugern ausgestattet werden. Dadurch entfallen bisher nötige aufwendige und störungsanfällige Zuleitungssysteme von einem zentralen Dampferzeuger mit den entsprechenden Steuerungsvorrichtungen für die Dampfströme. Die Gefahr der falschen Bedienung ist bei Anwendung des erfindungsgemäßen Verfahrens minimiert.

Erfindungsgemäß arbeitende Dampferzeuger finden weitere Einsatzgebiete in Dampfsterilisationsprozessen im Bereich der Pharmazie und in Verbindung mit speziellen Sterilisationsgeräten für die Entkeimung von Flüssigkeiten wie Milch oder Blut, mit denen der Wasserdampf in den Sterilisationsgeräten in direkten Kontakt tritt.

## Patentansprüche

1. Verfahren zum Sterilisieren mit Dampf, gekennzeichnet durch die nachstehenden Schritte:
- Einleiten von Wasserstoff und Sauerstoff in einen Dampferzeuger,
- Erzeugen von heißem Dampf durch Verbrennen des Wasserstoffs mit dem Sauerstoff in dem Dampferzeuger unter gleichzeitigem Einleiten von flüssigem Wasser in das heiße Verbrennungsgas, welches das Wasser zum Verdampfen bringt, und
- Einleiten des so erzeugten Dampfes in einen Behälter, in welchem sich das zu sterilisierende Gut befindet.

2. Verfahren nach Anspruch 1, dadurch gekennzeichnet, dass dem Wasserdampf der Dampf einer sterilisierend wirkenden Substanz zugegeben wird.

3. Verfahren nach Anspruch 1, dadurch gekennzeichnet, dass der Druck und die Temperatur des Dampfes gemessen und die Sterilisationsbedingungen gesteuert oder geregelt werden, indem einerseits die Zufuhr des Wasserstoffs und des Sauerstoffs und andererseits die Zufuhr des Wassers unabhängig voneinander in Abhängigkeit von dem gemessenen Druck und von der gemessenen Temperatur mengenmässig gesteuert oder geregelt werden.

4. Verfahren nach Anspruch 3, dadurch gekennzeichnet, dass der Dampfzustand zeitlich variabel gemäß einem vorgegebenen Programm gesteuert oder geregelt wird.

5. Verfahren nach einem der vorstehenden Ansprüche, dadurch gekennzeichnet, dass Wasserstoff und Sauerstoff in stöchiometrischem Verhältnis eingesetzt werden.

## Claims

1. A method of sterilizing by steam characterized by the subsequent steps:
- introducing hydrogen and oxygen into a steam generator,
- generating hot steam by burning the hydrogen with the oxygen in this steam generator while simultaneously introducing liquid water into the hot combustion gas which evaporates the water, and
- introducing the steam generated in this way into a vessel housing the goods which are to be sterilized.

2. A method according to claim 1, characterized in that the vapor of a sterilizing substance is added to the steam.

3. A method according to claim 1, characterized in that the pressure and the temperature of the steam are sensed and the sterilization conditions are controlled by controlling the mass supply of hydrogen and oxygen on the one hand and the mass supply of water on the other hand independently of each other responsive to the sensed pressure and to the sensed temperature.

4. A method according to claim 3, characterized in that the state of the steam is controlled in dependence on time in accordance with a predetermined program.

5. A method according to any of the preceding claims, characterized in that hydrogen and oxygen are supplied in a stoichiometric ratio.

## Revendications

1. Procédé de production de vapeur pour la stérilisation, caractérisé par les étapes suivantes :
- admission d'hydrogène et d'oxygène dans un générateur de vapeur,
- production de vapeur chaude par combustion de l'hydrogène avec l'oxygène dans le générateur de vapeur avec introduction simultanée d'eau liquide dans le gaz de combustion chaud, qui provoque la vaporisation de l'eau, et
- introduction de la vapeur ainsi produite dans un récipient qui contient le produit à stériliser.

2. Procédé selon la revendication 1, caractérisé en ce que la vapeur d'une substance agissant de manière stérilisante est ajoutée à la vapeur d'eau.

3. Procédé selon la revendication 1, caractérisé en ce que la pression et la température de la vapeur sont mesurées et les conditions de stérilisation sont commandées ou régulées du fait que, d'une part, l'admission de l'hydrogène et de l'oxygène et, d'autre part, l'admission de l'eau indépendamment l'une de l'autre, sont commandées ou régulées en fonction de la pression mesurée et de la température mesurée.

4. Procédé selon la revendication 3, caractérisé en ce que l'état de la vapeur est commandé ou régulé de manière variable dans le temps conformément à un programme prédéfini.

5. Procédé selon l'une quelconque des revendications précédentes, caractérisé en ce que l'hydrogène et l'oxygène sont utilisés dans un rapport stoechiométrique.
